# EUROPEAN PATENT SPECIFICATION

(11) **EP 4 044 972 B1**
(45) Date of publication and mention of the grant of the patent: **01.05.2024**
(21) Application number: 20801115.5
(22) Date of filing: 15.10.2020
(51) Int. Cl.: A61F 5/453, A61F 6/04

(54) **URINE COLLECTION DEVICE**
URINSAMMELVORRICHTUNG
DISPOSITIF DE COLLECTE D'URINE

(30) Priority: 18.10.2019 US 201962923279 P
(43) Date of publication of application: 24.08.2022
(73) Proprietor: Purewick Corporation, El Cajon, California 92020 (US)
(72) Inventor: JOHANNES, Ashley Marie, Atlanta, Georgia 30340 (US); CHALLA, Pranav, Atlanta, Georgia 30308 (US)
(74) Representative: Hoffmann Eitle
(86) International application number: PCT/US2020/055680
(87) International publication number: WO 2021/076695

(56) References cited:
- WO-A1-2006/021220
- CN-U- 202 184 840
- US-A- 3 998 228
- US-A- 5 423 784
- US-A- 5 592 950
- US-A1- 2017 354 532

## Description

### CROSS-REFERENCE TO RELATED APPLICATIONS

### BACKGROUND

An individual may have limited or impaired mobility such that typical urination processes are challenging or impossible. For example, the individual may have surgery or a disability that impairs mobility. In another example, the individual may have restricted travel conditions such as those experience by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters may be uncomfortable, painful, and may cause urinary tract infections.

Thus, users and manufacturers of fluid collection devices continue to seek new and improved devices, systems, and methods to collect urine. US 2017/0354532 A1 discloses a male catheter assembly comprising a braided section adapted to encompass a male penis and a sheath section connected to the braided section, the sheath section enabling connection to external devices such as a urine collection device. Holding a proximal end of the braided section at the base of the penis and gently pulling on the sheath section closes a spacing between the strands of the braid to constrict the braided section around the wearer's penis.

### SUMMARY

According to a first aspect of the invention, there is provided a fluid collection device according to claim 1, below. The dependent claims are directed to optional features and preferred embodiments.

Embodiments disclosed herein are related to fluid collection devices. In an embodiment, a fluid collection device is disclosed. The fluid collection device includes a tubular shaft and a receptacle. The tubular shaft has an open end and an inner surface. The tubular shaft is adjustable between a first configuration having a first inner circumference sized to receive at least a portion of a penis within the tubular shaft and a second configuration having a second inner circumference smaller than the first inner circumference and sized to contact the penis with the inner surface. The receptacle is connected to the tubular shaft distal to the open end. The receptacle is configured to collect urine discharged from the penis when the penis is positioned within the tubular shaft.

A method is disclosed. The method includes inserting a penis into a urine collection device such that a head of the penis is disposed within a receptacle of the urine collection device and a shaft of the penis is disposed within a tubular shaft of the urine collection device. The tubular shaft is cylindrical and has an open end and an inner surface, and the receptacle is connected to the tubular shaft distal to the open end of the tubular shaft. The method also includes adjusting the tubular shaft from a first configuration having a first inner circumference sized larger than a circumference of the shaft of the penis to a second configuration having a second inner circumference smaller than the first inner circumference and contacting the penis with the inner surface of the tubular shaft. The method also includes receiving urine discharged from the penis in the receptacle of the urine collection device. The method also includes removing the received urine from the receptacle of the urine collection device.

According to a second aspect of the invention, there is provided a method of manufacturing a fluid collection device according to claim 14.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1A** is an isometric view of a fluid collection device, according to an embodiment.
**FIG. 1B** is a cross-sectional view of the fluid collection device of **FIG. 1A** taken along the plane A-A, according to an embodiment.
**FIGS. 1C** is cross-sectional view of a strip of material of **FIG. 1A****,** according to an embodiment.
**FIG. 2A** is an isometric view of a fluid collection device, according to an embodiment.
**FIG. 2B** is a cross-sectional view of the fluid collection device of **FIG. 2A** taken along the plane B-B, according to an embodiment.
**FIG. 2C** is a cross-sectional view of a strip of material of **FIG. 2A****,** according to an embodiment.
**FIG. 3A** is an isometric view of a fluid collection device, according to an embodiment.
**FIG. 3B** is a cross-sectional view of the fluid collection device of **FIG. 3A** taken along plane C-C, according to an embodiment.
**FIG. 4A** is an isometric view of a fluid collection device, not presently claimed.
**FIG. 4B** is a cross-section view of the fluid collection device of **FIG. 4A** taken along plane D-D.
**FIG. 5** is a block diagram of a system for fluid collection, according to an embodiment.
**FIG. 6** is a flow diagram of a method of collecting fluids.

### DETAILED DESCRIPTION

Embodiments disclosed herein are related to fluid collection devices and methods of using the same. The devices and systems disclosed herein are configured to collect fluids from an individual. The fluids collected by the fluid collection devices may include at least one of urine, penile discharge, reproductive fluids, blood, sweat, or other bodily fluids.

The fluid collection devices have a generally cylindrical and expandable tubular shaft having an open end and an inner surface. The tubular shaft is adjustable between a first configuration and a second configuration. The first configuration of the tubular shaft includes a first inner circumference sized to receive at least a portion of a penis with the tubular shaft. In the first configuration, the tubular shaft is compressed longitudinally, thereby increasing the radius of the tubular shaft to allow insertion of the head and/or shaft of the penis into a central cavity of the tubular shaft. The second configuration of the tubular shaft includes a second inner circumference that is smaller than the first inner circumference and, in the second configuration, the second inner circumference is sized to contact the penis with the inner surface of the tubular shaft. In the second configuration, the tubular shaft is lengthened longitudinally, thereby decreasing the radius of the tubular shaft until the tubular shaft is secured to the shaft of the penis. The cylindrical and expandable tubular shaft allows the fluid collection device to be secured to the penis of a wearer without an adhesive or other separate securement device or strap. The cylindrical and expandable tubular shaft also allows the fluid collection device to be secured to the wearer without adhering or securing the device to the area around the penis of the wearer.

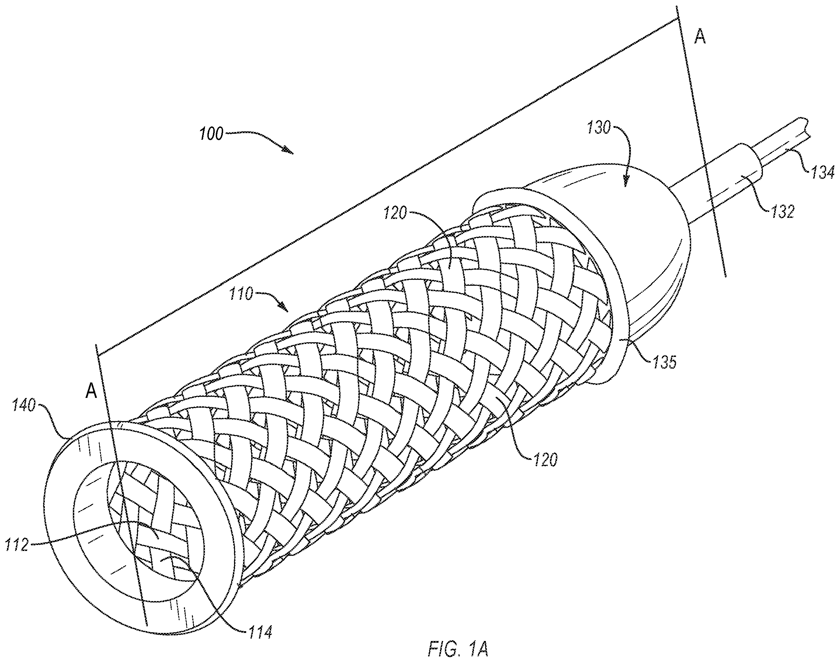

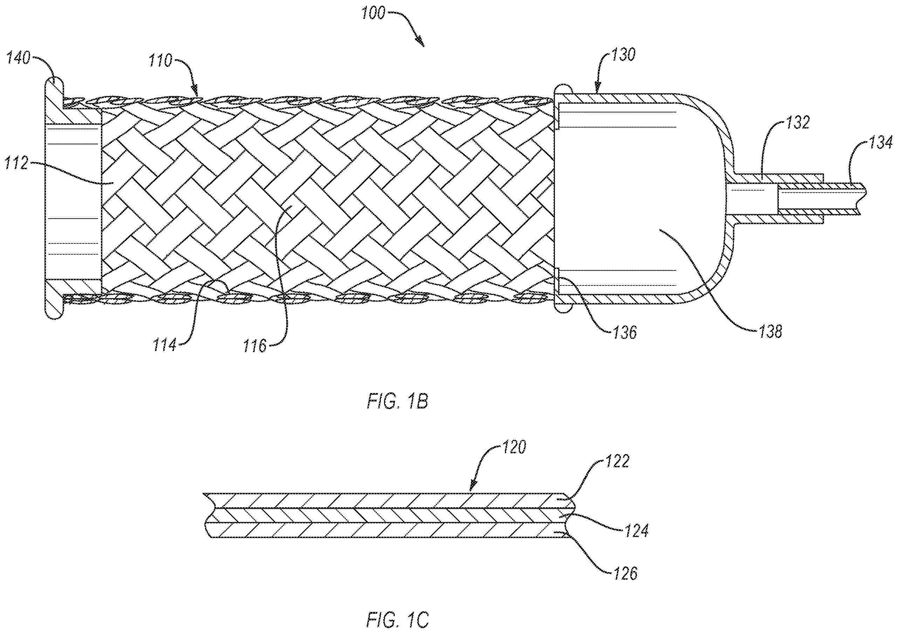

**FIG. 1A** is an isometric view of a fluid collection device 100, according to an embodiment. **FIG. 1B** is a cross-sectional view of the fluid collection device 100 of **FIG. 1A****,** taken along plane A-A. References to both **FIGS. 1A** and **1B** are made below. The fluid collection device 100 includes a tubular shaft 110 and a receptacle 130. The tubular shaft 110 includes an open end 112 and a cylindrical, helically wound braid of a plurality of strips 120 of one or more materials. The helically wound braid of the plurality of strips 120 form an inner surface 114 of the tubular shaft 110 that defines a central cavity 116 of the tubular shaft 110. The plurality of strips 120 may be interwoven in a cross-hatched helical pattern, thereby forming and defining the central cavity 116 of the tubular shaft 110.

The helically wound braid of the plurality of strips 120 allows the tubular shaft 110 to be adjustable and selectively expanded or retracted with respect to an inner diameter of the tubular shaft 110. For example, the tubular shaft 110 is adjustable between a first configuration or position and a second configuration or position. In the first configuration, the tubular shaft 110 includes a first diameter or first inner circumference that is sized to receive the head of a penis and at least a portion of a shaft of the penis therein, allowing the head of the penis to pass through the central cavity 116 of the tubular shaft 110 and into the receptacle 130. In the second configuration, the tubular shaft 110 includes a second diameter or second inner circumference that is smaller or less than the first diameter or first inner circumference, and also sized to contact the shaft of the penis with the inner surface 114 of the tubular shaft 110, thereby securing the tubular shaft 110 to the shaft of the penis. The second diameter or second inner circumference may vary according to the preference or anatomy of the wearer. In some embodiments, the tubular shaft 110 is biased to the second configuration in which the tubular shaft 110 is sized to contact the shaft of the penis. Accordingly, the tubular shaft 110 may remain in the second configuration in which the tubular shaft 110 is sized to contact the shaft of the penis unless acted upon by the wearer or caregiver adjusting the tubular shaft 110 to the first configuration.

In some embodiments, in the second configuration the tubular shaft 110 includes a longitudinal length of about 2 cm to about 22 cm, such as about 2 cm to about 12 cm, about 12 cm to about 22 cm, about 2 cm to about 7 cm, about 7 cm to about 12 cm, about 12 cm to about 17 cm, about 17 cm to about 22 cm, about 2 cm to about 4 cm, about 4 cm to about 6 cm, about 6 cm to about 8 cm, about 8 cm to about 10 cm, about 10 cm to about 12 cm, about 12 cm to about 14 cm, about 14 cm to about 16 cm, about 16 cm to about 18 cm, about 18 cm to about 20 cm, about 20 cm to about 22 cm, at least about 2 cm, at least about 5 cm, at least about 10 cm, at least about 15 cm, at least about 20 cm, about 2 cm, about 3 cm, about 4 cm, about 5 cm, about 6 cm, about 7 cm, about 8 cm, about 9 cm, about 10 cm, about 11 cm, about 12 cm, about 13 cm, about 14 cm, about 15 cm, about 16 cm, about 17 cm, about 18 cm, about 19 cm, about 20 cm, about 21 cm, or about 22 cm.

In some embodiments, in the second configuration the tubular shaft 110 includes an inner diameter of about 1.5 cm to about 8 cm, about 1.5 cm to about 4 cm, about 4 cm to about 8 cm, about 1.5 cm to about 2.5 cm, about 2 cm to about 3 cm, about 3 cm to about 4 cm, about 4 cm to about 5 cm, about 5 cm to about 6 cm, about 6 cm to about 7 cm, about 7 cm to about 8 cm, less than about 9 cm, less than about 8 cm, less than about 7 cm, less than about 6 cm, less than about 5 cm, less than about 4 cm, less than about 3 cm, or less than about 2 cm. In some embodiments, in the first configuration the tubular shaft 110 includes an inner diameter of about 5 cm to about 13 cm, about 5 cm to about 9 cm, about 9 cm to about 13 cm, about 5 cm to about 7 cm, about 7 cm to about 9 cm, about 9 cm to about 11 cm, about 11 cm to about 13 cm, about 5 cm to about 6 cm, about 6 cm to about 7 cm, about 7 cm to about 8 cm, about 8 cm to about 9 cm, about 9 cm to about 10 cm, about 10 cm to about 11 cm, about 11 cm to about 12 cm, about 12 cm to about 13 cm, at least about 5 cm, at least about 6 cm, at least about 7 cm, at least about 8 cm, at least about 9 cm, at least about 10 cm, at least about 11 cm, at least about 12 cm, or at least about 13 cm. The tubular shaft 110 may be adjustable between any of the dimensions provided above.

In some embodiments, the helically wound braid of the plurality of strips 120 includes a weaving having a sufficiently tight braid that substantially all fluids (*e.g.* urine) are prevented from leaking through the tubular shaft 110 when the tubular shaft 110 is in the second configuration and contacting the shaft of the penis. Preventing substantially all fluid from leaking through tubular shaft 110 may include preventing at least 90%, at least 95%, at least 99%, or 100% of fluid leaking through the tubular shaft 110 when the urine collection device 100 is worn by a wearer.

Each strip of the plurality of strips 120 in the helically wound braid may be configured to allow the strip to slide past one or more other strips of the plurality of strips 120 when the tubular shaft 110 expands or contracts. In some embodiments, such as the fluid collection device 100 in **FIG. 1A** and **1B****,** a gap or space may exist between adjacent helical strips 120. In some embodiments, adjacent helical strips 120 may generally interface or contact one another. Turning ahead to **FIG. 1C****,** in the fluid collection device 100, each strip of the plurality of strips 120 of one or more materials in the tubular shaft 110 includes a tri-layer strip of three materials. The tri-layer strip of three materials may include first or inner layer 122 of wicking material, a second or intermediate layer 124 of a pad material, and a third or outer layer 126 of a fluid impermeable material. Together, the first or inner layer 122 of the plurality of strips 120 may form the inner surface 114 of the tubular shaft 110, thereby at least partially defining the central cavity 116. The three materials in each strip may be held together with an adhesive, an ultrasonic weld, a heat weld, or stitching.

The first or inner layer 122 of wicking material includes a fluid permeable membrane that is configured to wick any fluid away from the open end 112 and the central cavity 116, thereby preventing fluid from escaping out of the open end 112 of the tubular shaft 110. The first or inner layer 122 also may wick any fluid in the tubular shaft 110 generally towards the receptacle 130, as discussed in more detail below. The first or inner layer 122 may include any material that can wick fluid. For example, the first or inner layer 122 can include fabric, such as gauze (*e.g*., a silk, linen, polyester, or cotton gauze), another soft fabric (*e.g*., jersey knit fabric or the like), or another smooth fabric (*e.g*., rayon, satin, or the like). In some examples, the first or inner layer 122 can include an open cell foam. Forming the first or inner layer 122 from gauze, soft fabric, and/or smooth fabric can reduce chafing caused by the fluid collection device 100. In some embodiments, the first or inner layer 122 may be omitted or absent from each strip of the plurality of strips 120. In some embodiments, the second or intermediate layer 124 and the third or outer layer 126 may be omitted or absent from each strip of the plurality of strips 120, and each strip of the plurality of strips 120 includes only the fluid permeable membrane of the first or inner layer 122.

The second or intermediate layer 124 may include a fluid permeable support layer configured to support the first or inner layer 122 since the first or inner layer 122 may be formed from a foldable, flimsy or otherwise easily deformable material. The second or intermediate layer 124 can be formed from any fluid permeable material that is less deformable than the first or inner layer 122. For example, the second or intermediate layer 124 can include a porous nylon structure or an open cell foam. In embodiments, the second or intermediate layer 124 may be omitted or absent from each strip of the plurality of strips 120. In some embodiments, the first or inner layer 122 and the third or outer layer 126 may be omitted or absent from each strip of the plurality of strips 120, and each strip of the plurality of strips 120 includes only the fluid permeable support of the second or intermediate layer 124.

The third or outer layer 126 may include a fluid impermeable layer configured to retain fluids in the fluid collection device 100. For example, the third or outer layer 126 may be formed of any suitable fluid impermeable materials, such as a fluid impermeable polymer (*e.g.*, silicone, polypropylene, polyethylene, polyethylene terephthalate (PET), polyurethane, a polycarbonate, a polyvinyl chloride, latex, silicone, etc.), a metal film, another suitable material, or combinations thereof. As such, the third or outer layer 126 may prevent at least some of the fluid(s) from exiting the tubular shaft 110 of the fluid collection device.

In some embodiments, the third or outer layer 126 may be air permeable and fluid impermeable. In such embodiments, the third or outer layer 126 may be formed of a hydrophobic material that defines a plurality of pores. In an example, one or more portions of at least an outer surface of the third or outer layer 126 may be formed from a soft and/or smooth material thereby reducing chafing of the skin of the user. In embodiments, the third or outer layer 126 may be omitted or absent from each strip of the plurality of strips 120.

Although each strip of the plurality of strips 120 in the fluid collection device 100 may include multiple layers of different materials, in some embodiments, the helically wound braid of the plurality of strips 120 may be formed from a single material. For example, the tubular shaft 110 can include a helically wound braid of a plurality of cords or strips 120 of a singular material, such as PET or any of the other materials referenced with respect to the first or inner layer 122, the second or intermediate layer 124, or the third or outer layer 126. In some embodiments, the tubular shaft 110 can include a helically wound braid of a plurality of strips 120 of a singular material that is different from any of the materials referenced with respect to the first or inner layer 122, the second or intermediate layer 124, or the third or outer layer 126

Returning to **FIGS. 1A** and **1B****,** the fluid collection device 100 also may include a ring 140 secured to the open end 112 of the tubular shaft 110. The ring 140 can be formed from a flexible polymer that exhibits a Young's modulus that is greater than the tubular shaft 110. As such, the ring 140 can provide some structure at or near the open end 112 of the tubular shaft 110. The increased rigidity of the ring 140 can cause the open end to remain open, thereby facilitating insertion of a penis into the fluid collection device 100. Further, in an embodiment, the increased rigidity of the ring 140 can enable the ring 140 to act as an attachment mechanism. In some embodiments, the ring 140 is omitted or absent from the fluid collection device 100.

The fluid collection device 100 also includes a receptacle 130 secured to the tubular shaft 110 distal to the open end 112 of the tubular shaft 110. The receptacle 130 may be secured to the tubular shaft 110 with at least one of an ultrasonic weld, a heat weld, an adhesive, stitching, etc. The receptacle 130 defines a chamber 138 within fluid collection device 100. The chamber 138 is in fluid communication with the central cavity 116 of the tubular shaft 110 when a penis is absent from the fluid collection device 100. The chamber 138 is sized and dimensioned to hold at least a portion of the head of the penis therein.

The receptacle 130 includes at least a fluid impermeable layer. In the fluid collection device 100 shown in **FIGS. 1A** and **1B****,** the receptacle 130 includes at least a head of a condom catheter. The receptacle 130 includes an outlet 132 configured connect to a tube 134 for drainage of urine from the urine collection device 100. The outlet 132 is generally positioned distal to the open end 112 of the tubular shaft 110. The receptacle 130 may include an elastic material the stretches or shrinks to secure to the head of the penis. In some embodiments, the receptacle 130 includes a roll 135 of material that may be rolled over at least a portion of the tubular shaft 110 after the penis is disposed within the fluid collection device 100.

The receptacle 130 may include a flexible or elastic material, such as polyurethane, resin, polyisoprene, latex, or combinations thereof. In some embodiments, the receptacle 130 includes one or more materials configured to retain a shape of the receptacle 130 when a penis is not positioned within the fluid collection device. In these and other embodiments, the receptacle 130 includes one or more of silicone, polypropylene, polyethylene, PET, polyurethane, a polycarbonate, a polyvinyl chloride, latex, silicone, etc. The receptacle 130 also may include an annular flap 136 between at least a portion of the receptacle 130 and the tubular shaft 110. The annular flap 136 may be a fluid impermeable flexible material that acts as seal between the chamber 138 and the central cavity 116 when a penis is inserted into the fluid collection device. In some embodiments, the annular flap 136 may be omitted from the fluid collection device 100.

In use, a penis is inserted through the open end 112 of the fluid collection device 100 at least partially into the tubular shaft 110. In some embodiments, the penis is inserted through the open end 112 of the fluid collection device 100 until the head of the penis passes through the tubular shaft 110 and is at least partially positioned within the chamber 138 of the receptacle 130. To insert the penis into the fluid collection device 100, the tubular shaft 110 may be compressed along a longitudinal axis, thereby increasing the inner circumference or diameter of the tubular shaft 110. In some embodiments, with the head of the penis at least partially positioned within the chamber 138 of the receptacle 130, the annular flap 136 may contact the penis and seal the chamber 138 from the central cavity 116 of the tubular shaft 110. In some embodiments, the receptacle 130 forms a seal between the chamber 138 and the central cavity 116 without the annular flap 136. In some embodiments, the weave of the tubular shaft 110 forms a seal that prevents fluids from exiting the fluid collection device 100 when the tubular shaft 110 is in the second configuration and contacting the penis positioned within the tubular shaft 110.

With the head of the penis positioned within the tubular shaft 110 or the chamber 138 of the receptacle 130, the tubular shaft 110 may be adjusted to the second configuration to secure the tubular shaft 110 to the shaft of the penis. As noted above, the tubular shaft 110 may be lengthened to decrease the inner circumference or diameter of the tubular shaft 110, thereby securing the tubular shaft to the penis without an adhesive. The tubular shaft 110 may be lengthened by pulling one end of the tubular shaft 110 away from the other end of the tubular shaft 110. The tubular shaft 110 also may be lengthened by compressing radially the tubular shaft 110. The adjustable tubular shaft 110 also allows a wearer to adjust the tubular shaft 110 to a desired comfortable tightness around the penis. Once the tubular shaft 110 is adjusted to tighten around the penis and the head of the penis is within the receptacle 130, urine discharged from the penis may exit through the outlet 132 of the receptacle 130, as described in greater detail below. The fluid collection device 100 may be fluidly coupled to a vacuum source that pulls or sucks discharged urine from the receptacle 130. In embodiments of the fluid collection device 100 having a plurality of strips 120 of three layers of material, urine in the tubular shaft 110 may be wicked from the tubular shaft 110 to the chamber 138 for removal from the urine collection device 100.

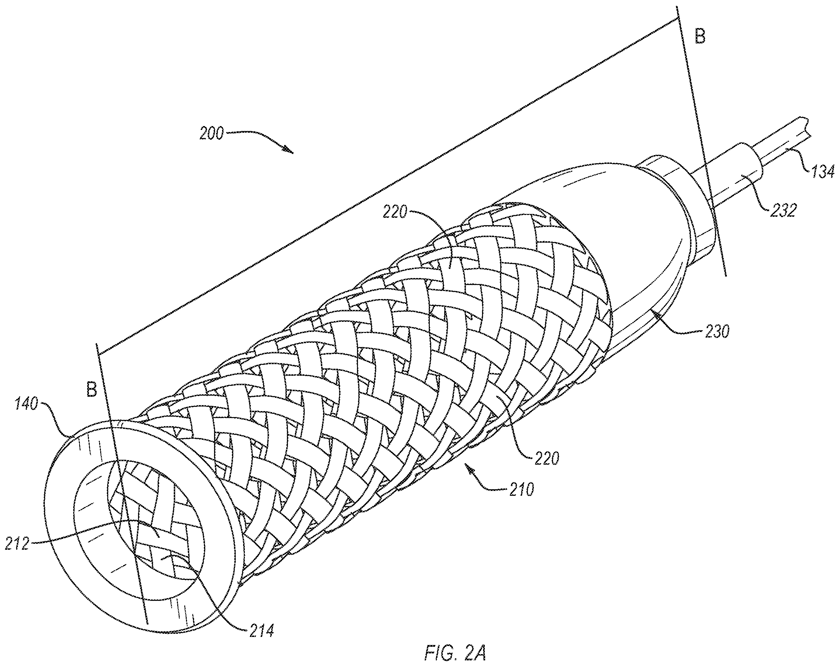

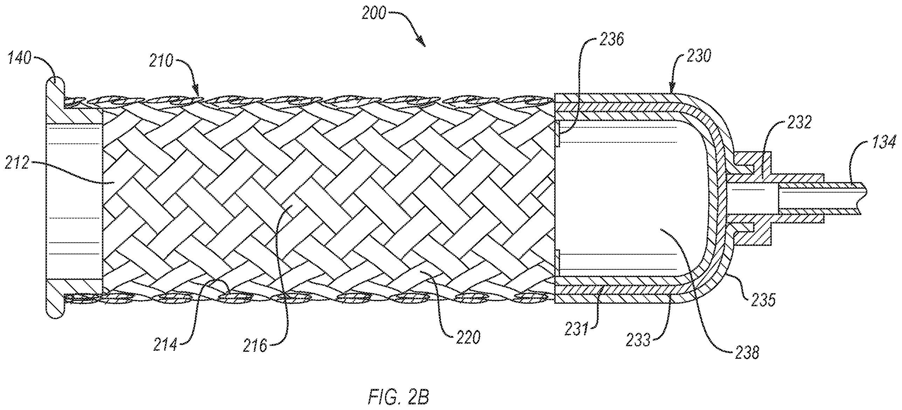

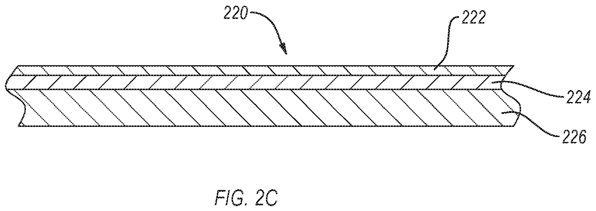

**FIG. 2A** is an isometric view of a fluid collection device 200, according to an embodiment. **FIG. 2B** is a cross-sectional view of the fluid collection device 200 of **FIG. 2A****,** taken along plane B-B. References to both **FIGS. 2A** and **2B** are made below. The fluid collection device 200 may include any of the features described above in relation to fluid collection device 100. For example, the fluid collection device 200 includes a tubular shaft 210 having an open end 212 and a cylindrical, helically wound braid of a plurality of strips 220 of one or more materials that form an inner surface 214 and define a central cavity 216. The helically wound braid of the plurality of strips 220 allows the tubular shaft 210 to be adjustable and selectively expanded or retracted with respect to an inner diameter of the tubular shaft 210, as described above in relation to the tubular shaft 110 of the fluid collection device 100. The tubular shaft 210 may include of the dimensions in the first configuration and/or the second configuration as provided above in relation to the tubular shaft 110. Turning to **FIG. 2C****,** each strip of the plurality of strips 220 in the tubular shaft 210 may include a first or inner layer 222, a support wire 224, and a third or outer layer 226. The first or inner layer 222 may include any of the materials, features, or characteristics described above in relation to the first or inner layer 122, the second or intermediate layer 124, or the third or outer layer 126 of the tubular shaft 110. The support wire 224 is embedded in the strip between the first or inner layer 122 and the third or outer layer 226. The support wire 224 is configured to provide support to the tubular shaft 210 to allow the tubular shaft 210 to retain a generally cylindrical shape. The support wire 224 may include a metal wire, a plastic wire, a rubber wire, or combinations thereof. The third or outer layer 226 may include any of the materials, features, or characteristics described above in relation to the first or inner layer 122, the second or intermediate layer 124, or the third or outer layer 126 of the tubular shaft 110. In some embodiments, at least one of the first or inner layer 222 or the third or outer layer 226 are omitted or absent from the plurality of strips 220. In some embodiments, the helically wound braid of the plurality of strips 220 includes a weave having a sufficiently tight braid that substantially all fluids (*e.g.* urine) are prevented from leaking through the tubular shaft 210 when the tubular shaft 210 is in the second configuration and contacting the shaft of the penis.

Returning to **FIGS. 2A** and **2B****,** the fluid collection device 200 also includes a receptacle 230 having an outlet 232. The receptacle 230 may be secured to the tubular shaft 210 with at least one of an ultrasonic weld, a heat weld, an adhesive, stitching, etc. The receptacle 230 includes first or inner layer 231 of wicking material, a second or intermediate layer 233 of a pad material, and a third or outer layer 235 of a fluid impermeable material. In some embodiments, at least one (*e.g*., both) of the first or inner layer 231 and the second or intermediate layer 233 cover at least a portion (*e.g*., all) of the outlet 232. In some embodiments, the first or inner layer 231 and the second or intermediate layer 233 do not cover at least a portion (*e.g*., all) of the outlet 232 such that the tube 134 has unrestricted fluid communication with the chamber 238.

The first or inner layer 231 at least partially defines a chamber 238 of the receptacle 230. The outlet 232 may be secured to at least one of the first or inner layer 230, the second or intermediate layer 233, or the third or outer layer 235 of the receptacle 230. The outlet 232 is configured to connect to the tube 134 for drainage of urine from the urine collection device 200. The receptacle 230 also may include an annular flap 236 between at least a portion of the receptacle 230 and the tubular shaft 210. The annular flap 230 may be a fluid impermeable flexible material that acts as seal between the chamber 238 and the central cavity 216 when a penis is inserted into the fluid collection device. The annular flap 236 may be secured to and/or extend from at least one of the first or inner layer 230, the second or intermediate layer 233, or the third or outer layer 235 of the receptacle 230. In some embodiments, the annular flap 236 may be omitted or absent from the fluid collection device 200.

The first or inner layer 231 of wicking material can include a fluid permeable membrane that is configured to wick any fluid away from the head of the penis and the chamber 238. The first or inner layer 231 also may wick any fluid in the tubular shaft 110 generally towards the outlet 232. The first or inner layer 231 may include any material that can wick fluid. For example, the first or inner layer 231 can include fabric, such as gauze (*e.g.,* a silk, linen, polyester, or cotton gauze), another soft fabric (*e.g*., jersey knit fabric or the like), or another smooth fabric (*e.g*., rayon, satin, or the like). In some examples, the first or inner layer 231 can include an open cell foam. Forming the first or inner layer 231 from gauze, soft fabric, and/or smooth fabric can reduce chafing cause by the fluid collection device 100. In some embodiments, the first or inner layer 231 may be omitted or absent from the receptacle 230.

The second or intermediate layer 233 may include a fluid permeable support layer configured to support the first or inner layer 231 since the first or inner layer 231 may be formed from a foldable, flimsy or otherwise easily deformable material. The second or intermediate layer 233 can be formed from any fluid permeable material that is less deformable than the first or inner layer 231. For example, the second or intermediate layer 233 can include a porous nylon structure or an open cell foam. In embodiments, the second or intermediate layer 233 may be omitted or absent from the receptacle 230.

The third or outer layer 235 may include a fluid impermeable layer. For example, the third or outer layer 235 may be formed of any suitable fluid impermeable materials, such as a fluid impermeable polymer (*e.g*., silicone, polypropylene, polyethylene, polyethylene terephthalate (PET), polyurethane, a polycarbonate, a polyvinyl chloride, latex, silicone, etc.), a metal film, another suitable material, or combinations thereof. As such, the third or outer layer 235 may prevent at least some of the fluid(s) from exiting the tubular shaft 110 of the fluid collection device.

In some embodiments, the third or outer layer 235 may be air permeable and fluid impermeable. In such embodiments, the third or outer layer 235 may be formed of a hydrophobic material that defines a plurality of pores. In an example, one or more portions of at least an outer surface of the third or outer layer 235 may be formed from a soft and/or smooth material thereby reducing chafing of the skin of the user. In some embodiments, the first or inner layer 231 and the second or intermediate layer 235 may be omitted from the receptacle 230, and the receptacle 230 includes only the fluid impermeable layer of the third or outer layer 230. In some embodiments, the receptacle 230 of the fluid collection device 200 may include any of the features described above in relation to the receptacle 130. In some embodiments, the receptacle 130 of the fluid collection device 100 (**FIG**. 1) may include any of the features described in relation to the receptacle 230.

The fluid collection device 200 may be used as described in relation to the fluid collection device 100. For example, a penis is inserted into the open end 212 of the fluid collection device 200 until the head of the penis is positioned in the tubular shaft 210 or passes through the tubular shaft 210 and is positioned within the chamber 238 of the receptacle 230. The tubular shaft 210 of the fluid collection device 200 may be adjusted to secure the tubular shaft 210 to the shaft of the penis as described in relation to the tubular shaft 110 of the fluid collection device 100.

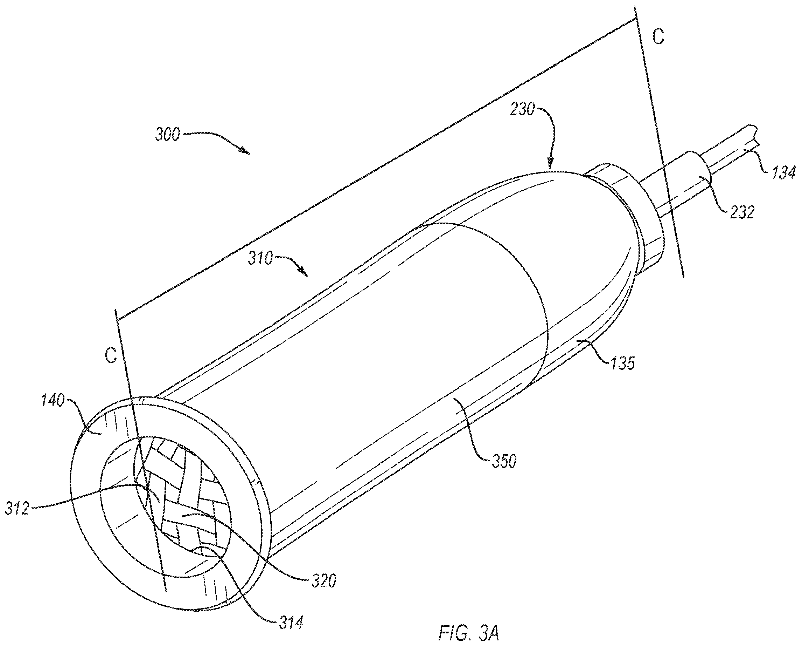

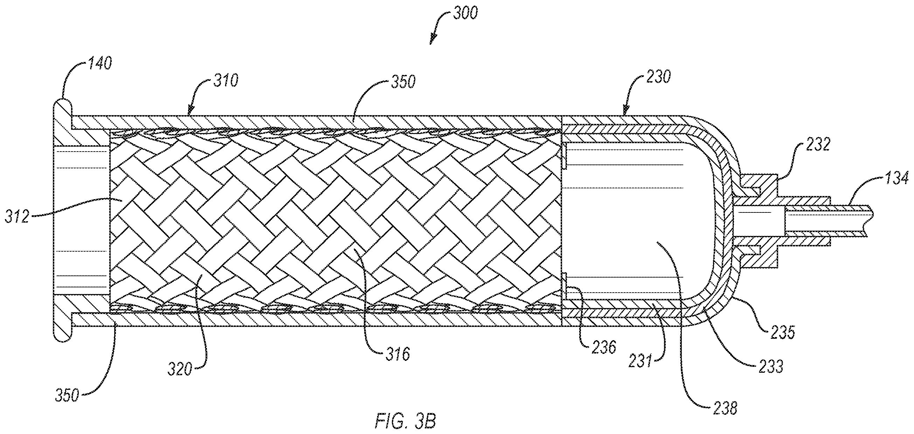

**FIG. 3A** is an isometric view of a fluid collection device 300, according to an embodiment. **FIG. 3B** is a cross-sectional view of the fluid collection device 300 of **FIG. 3A****,** taken along plane C-C. References to both **FIGS. 3A** and **3B** are made below. Unless otherwise noted, the fluid collection device 300 may include any of the features described above in relation to fluid collection devices 100 and 200. For example, the fluid collection device 300 includes a receptacle 230 having an outlet 232 configured to connect to a tube 134. Alternatively, the fluid collection device 300 may include a receptacle 130 described above.

The fluid collection device 300 also includes a tubular shaft 310 connected to the receptacle 230. The tubular shaft 310 includes an open end 312 and a cylindrical, helically wound braid of a plurality of strips 320 of one or more materials that form an inner surface 314 and define a central cavity 316 of the tubular shaft 310. The cylindrical, helically wound braid of a plurality of strips 320 of one or more materials may include any of the materials or features described in reference to the plurality of strips 120 or 220 of one or materials described above. The tubular shaft 310 may include of the dimensions in the first configuration and/or the second configuration as provided above in relation to the tubular shaft 110.

The plurality of strips 320 of one or more materials may be coated with a coating that prevents or inhibits urine from leaking through the helically wound braid of the plurality of strips 320. The coating may include at least one of a hydrophobic coating or a fluid impermeable coating. The coating may be applied to each strip of the plurality of strips 320 before the plurality of strips 320 are woven together to form the helically wound braid of the plurality of strips 320. Additionally or alternatively, the coating may be applied to the plurality of strips 320 after the plurality of strips 320 have been woven together to form the helically wound braid of the plurality of strips 320. For example, the tubular shaft 310 can include a helically wound braid of the plurality of strips 320 of a singular material, such as PET, that is coated with a hydrophobic coating or a fluid impermeable coating before or after the plurality of strips 320 of PET are woven together to form the helically wound braid of the plurality of strips 320. The helically wound braid of the plurality of strips 320 of the fluid collection device 300 also may be replace the helically wound braid of the plurality of strips 120 or 220 in the fluid collection devices 100 or 200, respectively.

The tubular shaft 310 also includes a fluid impermeable outer layer 350. The fluid impermeable outer layer 350 may be secured to opposing ends of the helically wound braid of the plurality of strips 320. The fluid impermeable outer layer 350 may include a collapsible material and a longitudinal length that is at least equal to a maximum longitudinal length of the helically wound braid of the plurality of strips 320. A fluid impermeable outer layer 350 having a longitudinal length that is at least equal to a maximum longitudinal length of the helically wound braid of the plurality of strips 320 allows the helically wound braid of the plurality of strips 320 to be elongated and retracted without interference from the fluid impermeable outer layer 350. In some embodiments, the fluid impermeable outer layer 350 includes an elastic or flexible material configured to elongate or retract with the helically wound braid of the plurality of strips 320. The outer layer 350 may be formed of any suitable fluid impermeable materials, such as a fluid impermeable polymer (*e.g*., silicone, polypropylene, polyethylene, polyethylene terephthalate (PET), polyurethane, a polycarbonate, a polyvinyl chloride, latex, silicone, etc.), a metal film, another suitable material, or combinations thereof. As such, the fluid impermeable outer layer 350 may prevent at least some of the fluid(s) from exiting the tubular shaft 310 of the fluid collection device 300. In some embodiments, the fluid impermeable outer layer 350 may be omitted from the fluid collection device 300. The fluid impermeable outer layer 350 also may be included in the fluid collection devices 100 and 200.

The fluid collection device 300 may be used as described in relation to the fluid collection devices 100 and 200. For example, a penis is inserted into the open end 312 of the fluid collection device 300 until the head of the penis is positioned within the tubular shaft 310 or passes through the tubular shaft 310 and is positioned within the chamber 238 of the receptacle 230. The tubular shaft 310 of the fluid collection device 300 may be adjusted to secure the tubular shaft 310 to the shaft of the penis as described in relation to the tubular shaft 110 of the fluid collection device 100.

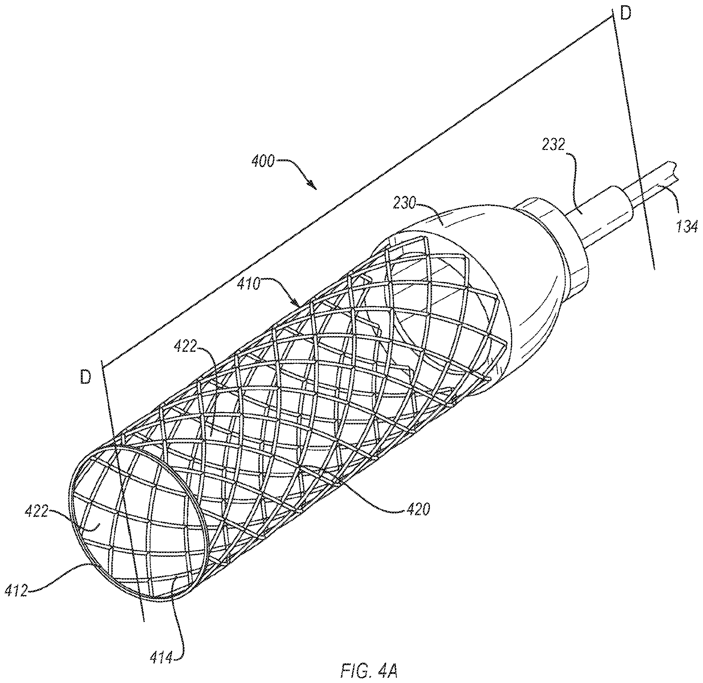

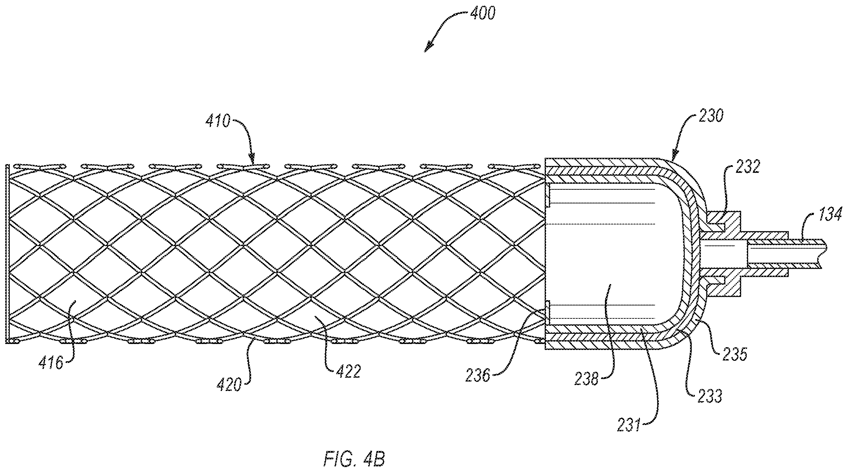

**FIG. 4A** is an isometric view of a fluid collection device 400, not presently claimed. **FIG. 4B** is a cross-sectional view of the fluid collection device 400 of **FIG. 4A****,** taken along plane D-D. References to both **FIGS. 4A** and **4B** are made below. Unless otherwise noted, the fluid collection device 400 may include any of the features described above in relation to fluid collection devices 100, 200, and 300. For example, the fluid collection device 400 includes a receptacle 230 having an outlet 232 configured to connect to a tube 134. Alternatively, the fluid collection device 400 may include a receptacle 130 described above.

The fluid collection device 400 also includes a tubular shaft 410 connected to the receptacle 230. The tubular shaft 410 includes an open end 412 and sleeve of netting 420 forming an inner surface 414 of the tubular shaft 410 and at least partially defining a central cavity 416 of the tubular shaft 410. The sleeve of netting 420 may include of a plurality of openings 422 extending therethrough. The plurality of openings 422 may be formed in a plurality of shapes that enhance the elasticity of the sleeve of netting 420, such as generally circular, oval, diamond-shaped, or other polygonal shapes. The tubular shaft 410 may include of the dimensions in the first configuration and/or the second configuration as provided above in relation to the tubular shaft 110.

The sleeve of netting 420 allows the tubular shaft 410 to be adjustable and selectively expanded or retracted with respect to an inner diameter of the tubular shaft 410. For example, the tubular shaft 410 is adjustable between a first configuration or position and a second configuration or position. In the first configuration, the tubular shaft 410 includes a first diameter or first inner circumference that is sized to receive the head of a penis and at least a portion of a shaft of the penis therein, allowing the head of the penis to pass through the central cavity 416 of the tubular shaft 410 and into the receptacle 230. In the second configuration, the tubular shaft 410 includes a second diameter or second inner circumference that is sized to contact the shaft of the penis with the inner surface 414 of the tubular shaft 410, thereby securing the tubular shaft 410 to the shaft of the penis. In some embodiments, the sleeve of netting is biased towards the second configuration.

In use, a penis is inserted through the open end 412 of the fluid collection device 400 until the head of the penis passes through the tubular shaft 410 and is positioned within the chamber 238 of the receptacle 230. The sleeve of netting 420 stretches to allow passage of the head of the penis and at least a portion of the shaft of the penis therethrough. The sleeve of netting 420 may be biased to the described second configuration, thereby contracting around at least a portion of the shaft of the penis to secure the fluid collection device 400 to the penis. Once the tubular shaft 410 is secured around the shaft of the penis and the head of the penis is within the receptacle 230, urine discharged from the penis may exit through the outlet 232 of the receptacle 230.

In some embodiments, with the head of the penis positioned within the chamber 238 of the receptacle 230, the annular flap 236 may contact the penis and seal the chamber 238 from the central cavity 416 of the tubular shaft 410. In some embodiments, the receptacle 230 forms a seal between the chamber 238 and the central cavity 416 without the annular flap 236. In some embodiments, the tubular shaft 410 may include an outer fluid impermeable layer secured to the tubular shaft 410, such as the outer layer 350 described above.

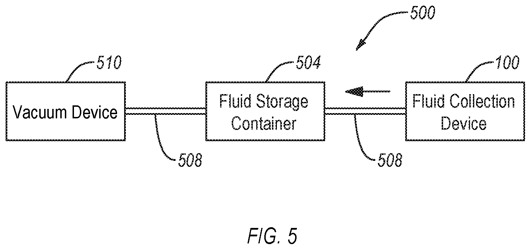

**FIG. 5** is a block diagram of a system 500 for fluid collection, according to an embodiment. The system 500 includes a fluid collection device 100. Although the fluid collection device 100 is shown in **FIG. 5****,** the system 500 includes any of the fluid collection devices 100, 200, 300. The fluid collection device 100 is fluidly coupled to a fluid storage container 504 through a conduit 508. At least a portion of the conduit 508 may include the tube 134 connected the fluid collection device 100. The fluid storage container 504 is fluidly coupled to a vacuum device 510 through a conduit 108. When activated, the vacuum device 510 is configured to pull a vacuum from the outlet 132 of the fluid collection device 100 through the fluid storage container 504. Accordingly, in use, the system 500 is configured to withdraw fluid, such as urine, from the fluid collection device 100 for storage within the fluid storage container 504. For example, as a user discharges urine in the fluid collection device, the vacuum device 510 pulls urine through the outlet 132 of the fluid collection device into the conduit 508 and into the fluid storage container 504.

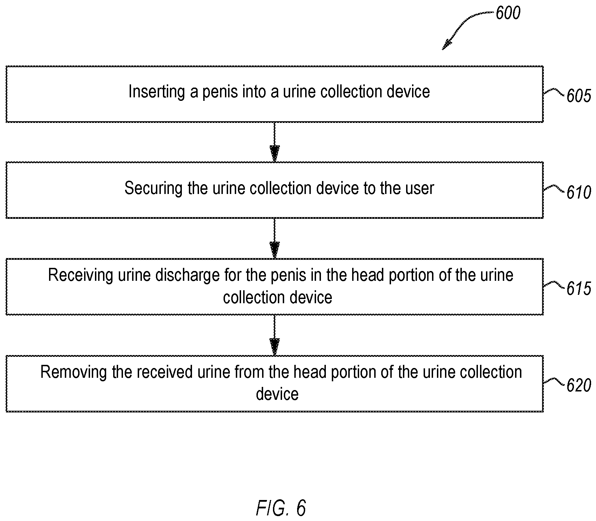

**FIG. 6** is a flow diagram of a method 600 for collecting fluids. The method 600 includes an act 605 of inserting a penis into a urine collection device. The act 605 may include inserting a penis into a urine collection device such that a head of the penis is disposed within a tubular shaft or a receptacle of the urine collection device and at least a portion of a shaft of the penis is disposed within the tubular shaft of the urine collection device. The tubular shaft of the urine collection device is cylindrical and has an open end and an inner surface, and the receptacle may be connected to the tubular shaft distal to the open end of the tubular shaft. The method 600 also includes an act 610 of securing the urine collection device to the user. The act 610 of securing the urine collection device to the user may include adjusting the tubular shaft from a first configuration having a first inner circumference sized larger than a circumference of the shaft of the penis to a second configuration having a second inner circumference smaller than the first inner circumference and contacting the penis with the inner surface of the tubular shaft. The method 600 also includes an act 615 of receiving urine discharged from the penis in the receptacle of the urine collection device. The method also includes an act 620 of removing the received urine from the receptacle of the urine collection device. In some embodiments, the method 600 also includes an act of fluidly coupling a vacuum source to a suction port on the receptacle of the urine collection device to assist in withdrawing the urine from the receptacle via the fluid outlet.

Acts 605, 610, 615, and 620 of the method 600 are for illustrative purposes. For example, the acts 605, 610, 615, and 620 of the method 600 can be performed in different orders, split into multiple acts, modified, supplemented, or combined. One or more of the acts 605, 610, 615, and 620 of the method 600 can be omitted from the method 600. Any of the acts 605, 610, 615, and 620 can include using any of the fluid collection devices or systems disclosed herein.

Also disclosed herein are one or more embodiments of a method of manufacturing a urine collection device. The urine collection device manufactured according to the method may include any urine collection devices 100, 200, or 300. In some embodiments, the method of manufacturing a urine collection device includes providing a tubular shaft having an open end and an inner surface, the tubular shaft being adjustable between a first configuration having a first inner circumference sized to receive at least a portion of a penis within the tubular shaft and a second configuration having a second inner circumference smaller than the first inner circumference and sized to contact the penis with the inner surface.

Providing a tubular shaft having an open end and an inner surface includes providing a cylindrical, helically wound braid of a plurality of a strips of one or more materials. More particularly, providing a cylindrical, helically wound braid of a plurality of a strips of one or more materials may include helically braiding the plurality of strips of one or more materials to form the cylindrical, helically wound braid of a plurality of a strips of one or more materials that is adjustable between the first configuration having the first inner circumference sized to receive at least a portion of the penis within the tubular shaft and the second configuration having the second inner circumference smaller than the first inner circumference and sized to contact the penis with the inner surface.

In some embodiments, the method may include securing a fluid impermeable layer radially outwardly to the cylindrical, helically wound braid of the plurality of strips of one or more materials of the tubular shaft. In some embodiments, the method of manufacturing a urine collection device may include securing a wicking material to the tubular shaft such that the wicking material defines at least a portion of the inner surface of the tubular shaft. In some embodiments, the method may include securing a padded material to the tubular shaft such that the padded material defines at least a portion of the inner surface of the tubular shaft. In some embodiments, the method of manufacturing a urine collection device may include securing a fluid impermeable layer positioned within the cylindrical, helically wound braid of the plurality of strips of one or more materials such that the fluid impermeable layer defines at least a portion of the inner surface of the tubular shaft.

In some embodiments, the method may include coating at least an outer surface of each strip of one or more materials of the plurality of strips of one or more materials with at least one of a hydrophobic coating or a fluid impermeable coating before the cylindrical, helically wound braid of the plurality of strips of one or more materials is formed. In some embodiments, the method o may include coating the cylindrical, helically wound braid of the plurality of strips of one or more materials with at least one of a hydrophobic coating or a fluid impermeable coating after the cylindrical, helically wound braid of the plurality of strips of one or more materials is formed. In some embodiments, the method of manufacturing a urine collection device may include securing one or more support wires to the tubular shaft to extend longitudinally through the cylindrical, helically wound braid of the plurality of strips of one or more materials.

The method may include connecting a receptacle to the tubular shaft distal to the open end, the receptacle being configured to collect urine discharged from the penis when the penis is positioned within the tubular shaft. In some embodiments, connecting a receptacle to the tubular shaft distal to the open end may include connecting the receptacle to the tubular shaft with at least one of an ultrasonic weld, a heat weld, or an adhesive.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

While various aspects and embodiments have been disclosed herein, other aspects and embodiments are contemplated. The various aspects and embodiments disclosed herein are for purposes of illustration and are not intended to be limiting. The scope of the invention is defined by the appended claims solely.

## Claims

1. A urine collection device (100;200;300), comprising:
a tubular shaft having an open end (112;212;312) and an inner surface defining a central cavity (116;216;316), the tubular shaft being adjustable between a first configuration having a first inner circumference sized to receive at least a portion of a penis within the tubular shaft and a second configuration having a second inner circumference smaller than the first inner circumference and sized to contact the penis with the inner surface, wherein the tubular shaft includes a cylindrical, helically wound braid of a plurality of a strips (120;220;320) of one or more materials, and wherein each of the plurality of strips includes a first layer (122;222) including a fluid permeable membrane configured to wick away fluid from the open end (112;212;312) and the central cavity (116;216;316); and
a receptacle (130;230;330) connected to the tubular shaft distal to the open end, the receptacle being configured to collect urine discharged from the penis when the penis is positioned within the tubular shaft;
**characterized in that**:
the tubular shaft includes a fluid impermeable layer (350) positioned radially outwardly to the cylindrical, helically wound braid of the plurality of strips of one or more materials; or
each strip (120;220;320) of one or more materials of the plurality of strips of one or more materials is coated on at least an outer surface with at least one of a hydrophobic coating or a fluid impermeable coating before the cylindrical, helically wound braid of the plurality of strips of one or more materials is formed; or
the cylindrical, helically wound braid of the plurality of strips of one or more materials is coated with at least one of a hydrophobic coating or a fluid impermeable coating after the cylindrical, helically wound braid of the plurality of strips of one or more materials is formed.

2. The urine collection device of claim 1, wherein the tubular shaft further includes a padded material defining at least a portion of the inner surface of the tubular shaft.

3. The urine collection device of claim 1, wherein the tubular shaft includes a fluid impermeable layer positioned within the cylindrical, helically wound braid of the plurality of strips of one or more materials and defining at least a portion of the inner surface of the tubular shaft.

4. The urine collection device of any of claims 1-3, wherein the tubular shaft further includes one or more support wires (224) extending longitudinally through the cylindrical, helically wound braid of the plurality of strips of one or more materials.

5. The urine collection device of any of claims 1-4, wherein at least one strip of the plurality of strips of one or more materials includes a longitudinally extending support wire (224).

6. The urine collection device of any of claims 1-5, wherein the first configuration includes a first longitudinal length and the second configuration includes a second longitudinal length that is greater than the first longitudinal length.

7. The urine collection device of any of claims 1-6, wherein the receptacle includes a fluid impermeable layer and a suction port.

8. The urine collection device of claim 7, wherein the receptacle further includes a wicking material defining at least a portion of an inner surface of the receptacle and a pad material positioned between the wicking material and the fluid impermeable layer.

9. The urine collection device of any of claims 1-8, wherein the receptacle is connected to the tubular shaft with at least one of an ultrasonic weld, a heat weld, or an adhesive.

10. The urine collection device of any of claims 1-9, further comprising an annular flange (236) positioned within the urine collection device and a chamber (238) sized to receive a head of the penis when the penis is positioned within the tubular shaft, the chamber being defined at least partially by a portion of the receptacle and a portion of the annular flange.

11. A method of manufacturing the urine collection device according to claim 1, the method comprising:
providing the tubular shaft; and
connecting the receptacle to the tubular shaft distal to the open end, the receptacle being configured to collect urine discharged from the penis when the penis is positioned within the tubular shaft.

## Patentansprüche

1. Urinsammelvorrichtung (100;200;300), umfassend:
einen rohrförmigen Schaft, der ein offenes Ende (112;212;312) und eine innere Oberfläche aufweist, die einen zentralen Hohlraum (116;216;316) definiert, wobei der rohrförmige Schaft zwischen einer ersten Konfiguration, die einen ersten inneren Umfang aufweist, der so bemessen ist, dass er zumindest einen Abschnitt eines Penis innerhalb des rohrförmigen Schafts aufnimmt, und einer zweiten Konfiguration verstellbar ist, die einen zweiten inneren Umfang aufweist, der kleiner als ist der erste innere Umfang und so bemessen ist, dass er den Penis mit der inneren Oberfläche berührt, wobei der rohrförmige Schaft ein zylindrisches, spiralförmig gewickeltes Geflecht einer Vielzahl von Streifen (120;220;320) aus einem oder mehreren Materialien einschließt, und wobei jeder der Vielzahl von Streifen eine erste Schicht (122;222) einschließt, die eine fluiddurchlässige Membran einschließt, die so konfiguriert ist, dass sie Fluid von dem offenen Ende (112;212;312) und dem zentralen Hohlraum (116;216;316) abtransportiert; und
einen Behälter (130;230;330), der distal zum offenen Ende mit dem rohrförmigen Schaft verbunden ist, wobei der Behälter so konfiguriert ist, dass er vom Penis abgegebenen Urin sammelt, wenn der Penis innerhalb des rohrförmigen Schafts positioniert ist;
**dadurch gekennzeichnet, dass**:
der rohrförmige Schaft eine fluidundurchlässige Schicht (350) einschließt, die radial außerhalb des zylindrischen, spiralförmig gewickelten Geflechts der Vielzahl von Streifen aus einem oder mehreren Materialien positioniert ist; oder
jeder Streifen (120;220;320) aus einem oder mehreren Materialien der Vielzahl von Streifen aus einem oder mehreren Materialien auf mindestens einer äußeren Oberfläche mit mindestens einer von einer hydrophoben Beschichtung oder einer fluidundurchlässigen Beschichtung beschichtet ist, bevor das zylindrische, spiralförmig gewickelte Geflecht der Vielzahl von Streifen aus einem oder mehreren Materialien gebildet wird; oder
das zylindrische, spiralförmig gewickelte Geflecht der Vielzahl von Streifen aus einem oder mehreren Materialien mit mindestens einer von einer hydrophoben Beschichtung oder einer fluidundurchlässigen Beschichtung beschichtet ist, nachdem das zylindrische, spiralförmig gewickelte Geflecht der Vielzahl von Streifen aus einem oder mehreren Materialien gebildet wird.

2. Urinsammelvorrichtung nach Anspruch 1, wobei der rohrförmige Schaft weiter ein gepolstertes Material einschließt, das mindestens einen Abschnitt der inneren Oberfläche des rohrförmigen Schafts definiert.

3. Urinsammelvorrichtung nach Anspruch 1, wobei der rohrförmige Schaft eine fluidundurchlässige Schicht einschließt, die innerhalb des zylindrischen, spiralförmig gewickelten Geflechts der Vielzahl von Streifen aus einem oder mehreren Materialien positioniert ist und mindestens einen Abschnitt der inneren Oberfläche des rohrförmigen Schafts definiert.

4. Urinsammelvorrichtung nach einem der Ansprüche 1-3, wobei der rohrförmige Schaft weiter einen oder mehrere Stützdrähte (224) einschließt, die sich in Längsrichtung durch das zylindrische, spiralförmig gewickelte Geflecht der Vielzahl von Streifen aus einem oder mehreren Materialien erstrecken.

5. Urinsammelvorrichtung nach einem der Ansprüche 1-4, wobei mindestens ein Streifen der Vielzahl von Streifen aus einem oder mehreren Materialien einen sich in Längsrichtung erstreckenden Stützdraht (224) einschließt.

6. Urinsammelvorrichtung nach einem der Ansprüche 1-5, wobei die erste Konfiguration eine erste Längslänge einschließt und die zweite Konfiguration eine zweite Längslänge einschließt, die größer als die erste Längslänge ist.

7. Urinsammelvorrichtung nach einem der Ansprüche 1-6, wobei der Behälter eine fluidundurchlässige Schicht und eine Saugöffnung einschließt.

8. Urinsammelvorrichtung nach Anspruch 7, wobei der Behälter weiter ein Dochtwirkungsmaterial, das mindestens einen Abschnitt einer inneren Oberfläche des Behälters definiert, und ein Polstermaterial einschließt, das zwischen dem Dochtwirkungsmaterial und der fluidundurchlässigen Schicht positioniert ist.

9. Urinsammelvorrichtung nach einem der Ansprüche 1-8, wobei der Behälter mit dem rohrförmigen Schaft mit mindestens einem von einer Ultraschallschweißung, einer Wärmeschweißung oder einem Klebstoff verbunden ist.

10. Urinsammelvorrichtung nach einem der Ansprüche 1-9, weiter umfassend einen ringförmigen Flansch (236), der innerhalb der Urinsammelvorrichtung positioniert ist, und eine Kammer (238), die so bemessen ist, dass sie einen Kopf des Penis aufnimmt, wenn der Penis innerhalb des rohrförmigen Schafts positioniert ist, wobei die Kammer zumindest teilweise durch einen Abschnitt des Behälters und einen Abschnitt des ringförmigen Flansches definiert ist.

11. Verfahren zum Herstellen der Urinsammelvorrichtung nach Anspruch 1, wobei das Verfahren umfasst:
Bereitstellen des rohrförmigen Schafts; und
Verbinden des Behälters mit dem rohrförmigen Schaft distal zum offenen Ende, wobei der Behälter so konfiguriert ist, dass er vom Penis abgegebenen Urin sammelt, wenn der Penis innerhalb des rohrförmigen Schafts positioniert ist.

## Revendications

1. Dispositif (100 ; 200 ; 300) de collecte d'urine, comprenant :
un arbre tubulaire présentant une extrémité ouverte (112 ; 212 ; 312) et une surface interne définissant une cavité centrale (116 ; 216 ; 316), l'arbre tubulaire étant réglable entre une première configuration présentant une première circonférence interne dimensionnée pour recevoir au moins une partie d'un pénis à l'intérieur de l'arbre tubulaire et une seconde configuration présentant une seconde circonférence interne plus petite que la première circonférence interne et dimensionnée pour mettre en contact le pénis avec la surface interne, dans lequel l'arbre tubulaire inclut une tresse cylindrique enroulée de manière hélicoïdale d'une pluralité de bandes (120 ; 220 ; 320) d'un ou de plusieurs matériaux, et dans lequel chacune de la pluralité de bandes inclut une première couche (122 ; 222) incluant une membrane perméable aux fluides configurée pour absorber le fluide de l'extrémité ouverte (112 ; 212 ; 312) et de la cavité centrale (116 ;216 ;316) ; et
un réceptacle (130 ; 230 ; 330) relié à l'arbre tubulaire distal par rapport à l'extrémité ouverte, le réceptacle étant configuré pour collecter l'urine évacuée du pénis lorsque le pénis est positionné à l'intérieur de l'arbre tubulaire ;
**caractérisé en ce que** :
l'arbre tubulaire inclut une couche imperméable aux fluides (350) positionnée radialement vers l'extérieur par rapport à la tresse cylindrique enroulée de manière hélicoïdale de la pluralité de bandes d'un ou plusieurs matériaux ; ou
chaque bande (120 ; 220 ; 320) d'un ou de plusieurs matériaux de la pluralité de bandes d'un ou plusieurs matériaux est revêtue sur au moins une surface externe d'au moins l'un d'un revêtement hydrophobe ou un revêtement imperméable aux fluides avant la formation de la tresse cylindrique enroulée de manière hélicoïdale de la pluralité de bandes d'un ou plusieurs matériaux ; ou
la tresse cylindrique enroulée de manière hélicoïdale de la pluralité de bandes d'un ou plusieurs matériaux est revêtue d'au moins l'un d'un revêtement hydrophobe ou un revêtement imperméable aux fluides après la formation de la tresse cylindrique enroulée de manière hélicoïdale de la pluralité de bandes d'un ou plusieurs matériaux.

2. Dispositif de collecte d'urine selon la revendication 1, dans lequel l'arbre tubulaire inclut en outre un matériau rembourré définissant au moins une partie de la surface interne de l'arbre tubulaire.

3. Dispositif de collecte d'urine selon la revendication 1, dans lequel l'arbre tubulaire inclut une couche imperméable aux fluides positionnée à l'intérieur de la tresse cylindrique enroulée de manière hélicoïdale de la pluralité de bandes d'un ou plusieurs matériaux et définit au moins une partie de la surface interne de l'arbre tubulaire.

4. Dispositif de collecte d'urine selon l'une quelconque des revendications 1 à 3, dans lequel l'arbre tubulaire inclut en outre un ou plusieurs fils (224) de support s'étendant longitudinalement dans la tresse cylindrique enroulée de manière hélicoïdale de la pluralité de bandes d'un ou plusieurs matériaux.

5. Dispositif de collecte d'urine selon l'une quelconque des revendications 1 à 4, dans lequel au moins une bande de la pluralité de bandes d'un ou plusieurs matériaux inclut un fil (224) de support s'étendant longitudinalement.

6. Dispositif de collecte d'urine selon l'une quelconque des revendications 1 à 5, dans lequel la première configuration inclut une première longueur longitudinale et la seconde configuration inclut une seconde longueur longitudinale qui est supérieure à la première longueur longitudinale.

7. Dispositif de collecte d'urine selon l'une quelconque des revendications 1 à 6, dans lequel le réceptacle inclut une couche imperméable aux fluides et un orifice d'aspiration.

8. Dispositif de collecte d'urine selon la revendication 7, dans lequel le réceptacle inclut en outre un matériau absorbant définissant au moins une partie d'une surface interne du réceptacle et un matériau tampon positionné entre le matériau absorbant et la couche imperméable aux fluides.

9. Dispositif de collecte d'urine selon l'une quelconque des revendications 1 à 8, dans lequel le réceptacle est relié à l'arbre tubulaire avec au moins l'un d'une soudure à ultrasons, une soudure thermique ou un adhésif.

10. Dispositif de collecte d'urine selon l'une quelconque des revendications 1 à 9, comprenant en outre une bride annulaire (236) positionnée à l'intérieur du dispositif de collecte d'urine et une chambre (238) dimensionnée pour recevoir une tête du pénis lorsque le pénis est positionné à l'intérieur de l'arbre tubulaire, la chambre étant définie au moins partiellement par une partie du réceptacle et une partie de la bride annulaire.

11. Procédé de fabrication du dispositif de collecte d'urine selon la revendication 1, le procédé comprenant :
la fourniture de l'arbre tubulaire ; et
la liaison du réceptacle à l'arbre tubulaire distal par rapport à l'extrémité ouverte, le réceptacle étant configuré pour collecter l'urine évacuée du pénis lorsque le pénis est positionné à l'intérieur de l'arbre tubulaire.
